(19) **Europäisches Patentamt**

**European Patent Office**

**Office européen des brevets**

(11) **EP 0 927 766 B1**

(12) **EUROPÄISCHE PATENTSCHRIFT**

(45) Veröffentlichungstag und Bekanntmachung des Hinweises auf die Patenterteilung:
**14.06.2006 Patentblatt 2006/24**

(51) Int Cl.:
*C12P 13/00* *(2006.01)*

(21) Anmeldenummer: **98124003.9**

(22) Anmeldetag: **17.12.1998**

(54) **Enzymatisches Verfahren zur Herstellung von (S)-Cyanhydrinen**

Enzymatic process for the preparation of (S)-cyanhydrines

Procédé enzymatique de préparation de (S)-cyanhydrines

(84) Benannte Vertragsstaaten:
**AT BE CH DE DK ES FR GB IT LI NL**

(30) Priorität: **29.12.1997 AT 219297**

(43) Veröffentlichungstag der Anmeldung:
**07.07.1999 Patentblatt 1999/27**

(73) Patentinhaber: **DSM Fine Chemicals Austria Nfg GmbH & Co KG**
**4021 Linz (AT)**

(72) Erfinder:
• **Pöchlauer, Peter**
**4040 Linz (AT)**
• **Schmidt, Michael**
**8113 St.Oswald (AT)**
• **Wirth, Irma**
**4470 Enns (AT)**
• **Neuhofer, Rudolf**
**4210 Mittertreffling (AT)**
• **Zabelinskaja-Mackova, Antonia A.**
**8020 Graz (AT)**
• **Griengl, Herfried**
**A-8047 Graz (AT)**
• **Van den Broek, Cor**
**6372 KX Landgraaf (NL)**
• **Reintjens, Raf W.E.G.**
**4871 CH Etten-Leur (NL)**
• **Wories, Herman Jelle**
**6215 EJ Maastricht (NL)**

(74) Vertreter: **Lindinger, Ingrid**
**DSM Fine Chemicals Austria GmbH**
**CEL/Patente**
**St. Peter-Strasse 25**
**4021 Linz (AT)**

(56) Entgegenhaltungen:
**EP-A- 0 547 655 EP-A- 0 632 130**
**US-A- 4 859 784**

EP 0 927 766 B1

## Beschreibung

Cyanhydrine sind etwa zur Synthese von alpha-Hydroxysäuren, alpha-Hydroxyketonen, beta-Aminoalkoholen, die zur Gewinnung biologisch wirksamer Stoffe, z. B. pharmazeutischer Wirkstoffe, Vitamine oder auch pyrethroider Verbindungen Verwendung finden, von Bedeutung.

Die Herstellung eines Cyanhydrins kann durch Anlagerung von Blausäure (HCN) an die Carbonylgruppe eines Aldehyds oder eines unsymmetrischen Ketons erfolgen, wobei Enantiomerengemische unsymmetrischer Cyanhydrine entstehen.

Da in einem biologisch wirksamen Enantiomerengemisch normalerweise nur eines der beiden Enantiomere biologisch wirksam ist, hat es nicht an Versuchen gefehlt, ein Verfahren zur Herstellung des (S)-Enantiomeren eines optisch aktiven Cyanhydrins in möglichst hoher optischer Reinheit zu finden.

So ist etwa in Makromol. Chem. 186, (1985), 1755-62 ein Verfahren zur Gewinnung von (S)-Cyanhydrinen durch Umsetzung von Aldehyden mit Blausäure in Gegenwart von Benzyloxycarbonyl-(R)-phenylalanin-(R)-histidinmethylester als Katalysator beschrieben. Die optische Reinheit der erhaltenen (S)-Cyanhydrine ist aber höchst unbefriedigend.

In EP-A-0 326 063 ist ein enzymatisches Verfahren zur Herstellung von optisch aktiven (R)- oder (S)-Cyanhydrinen durch Umsetzung von aliphatischen, aromatischen oder heteroaromatischen Aldehyden oder Ketonen mit Blausäure in Gegenwart von (R)-Oxynitrilase (EC 4.1.2.10) aus Prunus amygdalis oder Oxynitrilase (EC 4.1.2.11) aus Sorghum bicolor beschrieben. Beispiele für die stereospezifische Herstellung von aliphatischen (S)-Cyanhydrinen sind nicht angegeben. Dies ist nicht verwunderlich, denn in Angew. Chemie 102 (1990), Nr. 4, pp. 423 - 425 ist von Erfindern, die in EP-A-0 326 063 genannt sind, angegeben, daß von der (S)-Oxynitrilase aus Sorghum bicolor keine aliphatischen (S)-Cyanhydrine mit Blausäure hergestellt werden können. Dieser Befund wird durch F. Effenberger et al. in Tetrahedron Letters Vol. 31 No. 9 (1990), pp. 1249 -1 252 bestätigt.

EP 0 632 130 beschreibt weiters ein Verfahren bei welchem aliphatische Aldehyde oder unsymmetrische aliphatische Ketone mit Blausäure und Oxynitrilase aus Hevea brasiliensis stereospezifisch zu (S)-Cyanhydrinen umgesetzt werden. Die Umsetzung erfolgt gemäß EP 0 632 130 bevorzugt in einem wässrigen Verdünnugsmittel ohne Zusatz organischer Lösungsmittel, da diese, wie in EP 0 632 130 beschrieben, die Aktivität des Enzyms rasch hemmen.

EP 0 547 655 und US 4,859 784 beschreiben Verfahren, bei welchen optisch aktive Cyanhydrine aus Aldehyden oder Ketonen und Blausäure in einem Zweiphasensystem, bestehend aus einer homogenen wässrigen Lösung der Hydroxynitrilase und einem geeigneten organischen Lösungsmittel, das zumindest im mit Wasser Wesentlichen unmischbar ist, hergestellt werden. Das Reaktionssystem wird während der enzymatischen Umsetzung gerührt, wobei das Zweiphasensystem erhalten bleibt.

Bei den bisher bekannten Verfahren wurde zumeist ziemlich verdünnt, entweder in einem wäßrigen oder organischen System oder im Zweiphasensystem gearbeitet. Diese Verfahrensweise hat jedoch für viele Edukte Nachteile. So sind beispielsweise 3-Phenoxybenzaldehyd oder 4-Fluor-3-phenoxybenzaldehyd oder diverse Ketone schlechte Substrate, sodaß ein hoher Enzymeinsatz notwendig ist, um eine akzeptable Ausbeute an Cyanhydrinen mit guter optischer Reinheit zu erhalten.

Es wurde nun unerwarteterweise gefunden, daß die Umsetzung einer Vielzahl von Carbonylverbindungen, wie etwa aliphatische, alicyclische, ungesättigte, aromatisch substituierte aliphatische, aromatische, sowie heteroaromatische Aldehyde und Ketone, zu den entsprechenden Cyanhydrinen mit hoher Ausbeute und hoher optischer Reinheit in gegenüber dem Stand der Technik konzentrierterer Fahrweise und mit geringerem Enzymeinsatz möglich ist, wenn die Reaktion in einer Emulsion durchgeführt wird. Unerwarteterweise bleibt die Enzymaktivität unter den Bedingungen einer Emulsion, die bei vielen Proteinen zur Desaktivierung führen, wie etwa hoher Rührenergie, stabil.

Gegenstand der vorliegenden Anmeldung ist daher ein Verfahren zur Herstellung der (S)-Enantiomeren von optisch aktiven Cyanhydrinen durch Umsetzung eines Aldehyds oder eines Ketons mit einem Cyanidgruppendonor in Anwesenheit einer nativen oder einer rekombinanten (S)-Hydroxynitrillyase, das dadurch gekennzeichnet ist, daß ein Reaktionsgemisch aus

a) einem in einem organischen, mit Wasser nicht oder geringfügig mischbaren Verdünnungsmittel gelöster Aldehyd oder Keton,
b) einer wäßrigen (SI-Hydroxynitrillyaselösung und
c) einem Cyanidgruppendonor

mit einer Rührenergie über 500W/m$^3$ gerührt wird, daß eine Emulsion entsteht, die bis zum Reaktionsende durch Rühren aufrecht erhalten wird, worauf nach beendeter Reaktion das entsprechende (S)-Cyanhydrin aus dem Reaktionsgemisch durch Phasentrennung isoliert wird.

Als Ausgangsmaterialien im erfindungsgemäßen Verfahren werden ein Aldehyd oder ein Keton, ein Cyanidgruppendonor, eine wäßrige Lösung einer nativen oder rekombinanten Hydroxynitrillyase (Hnl) und ein organisches, mit Wasser nicht oder geringfügig mischbares Verdünnungsmittel eingesetzt.

Unter Aldehyden sind dabei aliphatische, aromatische oder heteroaromatische Aldehyde zu verstehen. Unter aliphatischen Aldehyden sind dabei gesättigte oder ungesättigte aliphatische, geradkettige, verzweigte oder cyclische Aldehyde zu verstehen. Bevorzugte aliphatische Aldehyde sind geradkettige Aldehyde mit insbesondere 2 bis 18 C-Atomen, bevorzugt von 2 bis 12, die gesättigt oder ein- oder mehrfach ungesättigt sind. Der Aldehyd kann dabei sowohl C-C-Doppelbindungen als auch C-C-Dreifachbindungen aufweisen. Der Aldehyd kann unsubstituiert oder durch unter den Reaktionsbedingungen inerte Gruppen, beispielsweise durch gegebenenfalls substituierte Aryl- oder Heteroarylgruppen wie Phenyl- oder Indolylgruppen, durch Halogen-, Ether-, Alkohol-, Acyl-, Carbonsäure-, Carbonsäureester-, Nitro- oder Azidogruppen substituiert sein. Beispiele für aromatische oder heteroaromatische Aldehyde sind Benzaldezyd bzw. verschieden substituierte Benzaldehyde wie etwa 3,4-Difluorbenzaldehyd, 3-Phenoxybenzaldehyd, 4-Fluor-3-phenoxybenzaldehyd, weiters Furfural, Anthracen-9-carbaldehyd, Furan-3-carbaldehyd, Indol-3-carbaldehyd, Naphthalin-1-carbaldehyd, Phthaldialdehyd, Pyrazol-3-carbaldehyd, Pyrrol-2-carbaldehyd, Thiophen-2-carbaldehyd, Isophthalaldehyd oder Pyridinaldehyde u.s.w..

Ketone sind aliphatische, aromatische oder heteroaromatische Ketone, bei denen das Carbonylkohlenstoffatom ungleich substituiert ist. Unter aliphatischen Ketonen sind gesättigte oder ungesättigte, geradkettige, verzweigte oder cyclische Ketone zu verstehen. Die Ketone können gesättigt oder ein- oder mehrfach ungesättigt sein. Sie können unsubstituiert, oder durch unter Reaktionsbedingungen inerte Gruppen, beispielsweise durch gegebenenfalls substituierte Aryl- oder Heteroarylgruppen wie Phenyl- oder Inolylgruppen, durch Halogen-, Ether-, Alkohol-, Acyl-, Carbonsäure-, Carbonsäureester-, Nitro- oder Azidogruppen substituiert sein.

Beispiele für aromatische oder heteroaromatische Ketone sind Acetophenon, Indolylaceton u.s.w..

Aldehyde und Ketone, die sich für das erfindungsgemäße Verfahren eignen, sind bekannt oder wie üblich herstellbar.

Als Cyanidgruppendonor wird Blausäure zugesetzt. Die Blausäure kann dabei auch erst kurz vor der Reaktion aus einem ihrer Salze wie etwa NaCN oder KCN freigesetzt und in Substanz oder in gelöster Form dem Reaktionsgemisch zugegeben werden.

Als Hydroxynitrillyase (Hnl) kommen native (S)-Hydroxynitrillyasen z.B. aus Maniok und Hevea brasiliensis, sowie rekombinante (S)-Hnl in Frage. Bevorzugt wird als native Hnl Hnl aus Hevea brasiliensis oder Manihot esculenta verwendet. Geeignete rekombinante (S)-Hnl wird beispielsweise aus gentechnisch modifizierten Mikroorganismen wie etwa Pichia pastoris oder Saccharomyces cerevisiae erhalten.

Bevorzugt wird rekombinante (S)-Hnl aus Pichia pastoris eingesetzt.

Durch die funktionelle Überexpression in der methylotrophen Hefe Pichia pastoris kann diese Hnl in beliebiger Menge gewonnen werden. (M.Hasslacher et al., J. Biol. Chem. 1996, 271, 5884) Dieses Expressionssystem eignet sich besonders für Fermentationen mit hoher Zelldichte. So ist es möglich, etwa 20 g reines Enzym pro Liter Fermentationsmedium zu erhalten. Die erreichbaren spezifischen Aktivitäten des gereinigten rekombinanten Proteins sind etwa doppelt so hoch als jene des natürlichen Enzyms, welches aus den Blättern des Baumes Hevea brasiliensis isoliert wurde. Nach dem Zellaufschluß kann die cytosolische Fraktion ohne weitere Reinigung verwendet werden, wodurch der Arbeitsaufwand minimiert wird. Das Enzym ist nicht glykosyliert und besitzt auch keine prosthetische Gruppe, die bei Abspaltung vom Proteinteil zur Inaktivierung führen würde.

Die Hnl ist bei Raumtemperatur mehrere Tage ohne signifikanten Aktivitätsverlust einsetzbar, und ist bei -20°C ausreichend langzeitstabil. Dadurch ergibt sich die Möglichkeit der mehrmaligen Verwendung der selben Enzymcharge. Das Enzym zeichnet sich auch durch eine hohe Beständigkeit gegenüber Lösungsmitteln aus. Daher besteht die Möglichkeit, für die enzymatische Reaktion verschiedene organische Solventien einzusetzen, die die Bildung einer Emulsion erlauben, was sich günstig auf die Produktivität des jeweiligen Prozesses auswirkt.

Die Hydroxynitrillyase kann gereinigt oder ungereinigt, als solche oder immobilisiert eingesetzt werden. Die Bereitstellung und Reinigung der Hydroxynitrillyase kann beispielsweise durch Fällung mit Ammoniumsulfat und anschließender Gelfiltration, etwa gemäß D. Selmar et al., Physiologia Plantarum 75 (1989), 97-101 erfolgen.

Als organische Verdünnungsmittel können mit Wasser nicht oder geringfügig mischbare aliphatische oder aromatische Kohlenwasserstoffe, die gegebenenfalls halogeniert sind, Alkohole, Ether oder Ester oder Gemische davon verwendet werden.

Bevorzugt werden Methyl- tert. Butylether (MTBE), Diisopropylether, Dibutylether und Ethylacetat oder ein Gemisch aus MTBE und Toluol eingesetzt.

Pro g Aldehyd oder Keton werden etwa 0,1 bis 10 g Verdünnungsmittel und 10 bis 10 000 IU Aktivität Hydroxynitrillyase, bevorzugt etwa 50 bis 2000 IU zugesetzt.

Ein IU (International Unit) bezeichnet diejenige Menge eines Enzympräparates, die die Bildung von einem Mikromol Produkt pro Minute katalysiert. Die benötigte Menge der jeweiligen Hydroxynitrillyase ermittelt man am besten in einem Aktivitätstest, etwa gemäß Selmar et al., Analytical Biochemistry 166 (1987), 208-211.

Pro Mol eingesetzte Aldehyd- oder Ketogruppe werden mindestens 1 Mol, bevorzugt 1 bis 5 Mole, besonders bevorzugt 1 bis 2 Mole Cyanidgruppendonor zugegeben.

Bei dem erfindungsgemäßen Verfahren liegt der Aldehyd oder das Keton gelöst in dem organischen Verdünnungsmittel vor. Zu dieser Lösung wird das Enzym in Form einer wäßrigen Pufferlösung zugesetzt. Der pH-Wert dieser Lösung soll dabei unter 7, bevorzugt zwischen 3 und 6,5 liegen.

Das so erhaltene Reaktionsgemisch wird bei Temperaturen von 0° bis etwa 30°C derart gerührt, daß eine Emulsion entsteht. Die dafür notwendige Rührerdrehzahl (N) hängt dabei von der sogenannten Vermögenskennzahl des eingesetzten Rührers (Po), dessen Durchmesser (d), dem Reaktionsvolumen (V) sowie der Dichte (p) des Reaktionsmediums ab. Aus diesen Faktoren läßt sich die Rührenergie, d. h. die Rührerleistung pro Reaktionsvolumen (Volumen des Reaktionsgemisches, nicht der Apparatur) ableiten.

$$P/V = \frac{P_0 \cdot \rho \cdot N^3 \cdot d^5}{V}$$

Bevorzugt liegt die Rührenergie bei dem erfindungsgemäßen Verfahren über 500 W/m$^3$, besonders bevorzugt über 1000 W/m$^3$. Vergleichsweise werden bei bisher bekannten Verfahren, die etwa im wäßrigen, organischen oder zweiphasigen System arbeiten lediglich Rührenergien von etwa 100 W/m$^3$ erzielt.

Liegt das Reaktionsgemisch als Emulsion vor, so wird der Cyanidgruppendonor zugesetzt. Die Emulsion wird bis zum Reaktionsende durch Rühren aufrecht erhalten. Der Reaktionsverlauf kann dabei beispielsweise photometrisch über die Abnahme des Aldehydgehaltes verfolgt werden.

Je nach Edukt wird bei der Wellenlänge gemessen, bei der das Edukt absorbiert und das entstehende Cyanhydrin nicht. Die Absorption des Reaktionsgemisches sinkt somit mit steigender Conversion proportional.

Bei Verwendung eines Salzes der Blausäure kann zuerst die Blausäure aus einer Lösung des Salzes durch Zugabe von beispielsweise $H_2SO_4$ oder $H_3PO_4$, freigesetzt werden.

Der pH-Wert dieser Lösung von Blausäure sollte unter 7, bevorzugt zwischen 4 und 6,5 liegen.

Anschließend wird zu der Blausäurelösung die wäßrige Enzymlösung, das organische Verdünnungsmittel und der Aldehyd oder das Keton zugegeben, die Reaktion gestartet und gegebenenfalls der pH-Wert nachjustiert.

Auch hier ist wieder darauf zu achten, daß das Reaktionsgemisch derart gerührt wird, daß eine Emulsion entsteht, die wiederum bis zum Ende der Reaktion durch kontinuierliches Rühren aufrecht erhalten wird.

Zur Aufarbeitung des Reaktionsgemisches und zur Isolierung des gebildeten Cyanhydrines werden übliche Techniken, welche zunächst die Emulsion brechen, wie etwa Filtration, Zentrifugation oder Koaleszierung eingesetzt. Anschließend werden die sich bildenden Phasen gegebenenfalls unter Zugabe von Demulgatoren aufgetrennt und die produkthaltige Phase aufgearbeitet.

Zur Gewinnung des entsprechenden Cyanhydrines werden dabei je nach Endprodukt bekannte Techniken wie Destillation, Extraktion oder Kristallisation angewandt. Die so gewonnenen Cyanhydrine können gegebenenfalls durch Zugabe einer Säure vor der Weiterverarbeitung stabilisiert werden.

Beispiel 1 - 8: Herstellung von (S)-3-Phenoxybenzaldehyd-cyanhydrin

Beispiel 1

a) 15 g (0,0756 mol) m-Phenoxybenzaldehyd wurden in 45 ml MTBE gelöst, in ein 150 ml Doppelmantelgefäß mit 6-Blattrührer eingebracht und die Reaktionsmischung auf 15°C gebracht.

26,4 ml Enzym (Hnl aus Pichia pastoris, Überstand der Zentrifugation des unverdünnten Zellysates) wurden in eine Lösung aus 1,5 g Sorbitol in 33,6 ml demineralisiertem Wasser (pH 5,8) gemischt, mit verdünnter Citronensäurelösung auf pH 5,5 gestellt und in den Reaktor eingebracht. Anschließend wurden 3 x 5 ml HCN über eine Spritze mit 3-Weghahn zugegeben. Die Reaktionslösung wurde dabei bereits mit 600 rpm gerührt.

Während der Reaktion wurde mit 800 rpm gerührt, sodaß das Reaktionsgemisch in Form einer Emulsion vorlag. Der Reaktionsverlauf wurde mittels IP-Kontrolle über die Abnahme des Aldehydgehaltes verfolgt. (Extinktion bei 304 nm) Dazu wurden 0,5 bis 1 ml Reaktionslösung nach definierten Reaktionszeiten entnommen und zentrifugiert. 100 $\mu$l organische Phase wurden mit MTBE auf 10 ml verdünnt, wovon wiederum 250 $\mu$l mit MTBE auf 10 ml verdünnt wurden, sodaß die Gesamtverdünnung einem Faktor von 100 x 40 = 4000 entsprach.

Gegen Ende der Reaktion (Conversion >95 %) wurde die Extinktion sehr klein, sodaß zur Steigerung der Genauigkeit der 2. Verdünnungsschritt entfiel, die Extinktion wurde in diesem Fall vor Berechnung der Conversion durch den 2. Verdünnungsfaktor (40) dividiert.

$$\text{Conversion}(t) = [1-(\text{Extinktion }(t) / \text{Extinktion (Start)})] \times 100\,\%$$

Folgende Conversionen wurden berechnet:

| Reaktionszeit | Conversion |
|---|---|
| 0 | 0 |
| 30 | 70 |
| 60 | 85 |
| 90 | 95 |
| 120 | 95... Extinktion bereits sehr klein, Reaktion bleibt stehen |

(Leitfähigkeit am Ende der Reaktion 0,8 mS)

b) Obiger Reaktionssatz wurde wiederholt, es wurde jedoch mit 1000 rpm gerührt.

| Reaktionszeit | Conversion |
|---|---|
| 0 | 0 |
| 60 | 90 |
| 90 | > 95 |
| 120 | > 95...Extinktion bereits sehr klein, lt. Dünnschichtchromatographie (DC) findet Reaktion bis zu einem Umsatz von 98 % statt. |

(Leifähigkeit am Ende der Reaktion 0,7 mS)

c) Zum Vergleich wurde obiger Ansatz ein weiteres Mal wiederholt, es wurde jedoch nur mehr mit 400 rpm gerührt.

| Reaktionszeit | Conversion |
|---|---|
| 0 | 0 |
| 30 | 30 |
| 60 | 70 |
| 90 | 75 |

(Leitfähigkeit am Ende der Reaktion 0,8 mS)

Die Rührenergie der 3 Ansätze wurde gemäß folgender Formel ermittelt:

$$P/V = \frac{P_0 \cdot \rho \cdot N^3 \cdot d^5}{V}$$

$P_0$     Vermögenskennzahl des Rührers
$\rho$     Dichte [kg/m$^3$]
N     Rührerdrehzahl [rpm]

d    Rührerdurchmesser [m]
V    Reaktionsvolumen [$m^3$]

| Beispiel 1 | rpm | Rührenergie [$W/m^3$] | |
|---|---|---|---|
| a | 800 | 588 | $P_0$.....3 |
| b | 1000 | 1146 | d.....0,5 |
| c | 400 | 73 | V............134 ml |

Beispiel 2

Analog Beispiel 1 wurden 75 g (0,378 mol) m-Phenoxybenzaldehyd in 225 ml MTBE und 132 ml Enzymlösung mit 73 ml (1,89 mol) HCN in einem 750 ml Doppelmantelgefäß mit 6 Blattrührer umgesetzt. Um eine Emulsion zu erhalten wurde dabei mit 770 rpm, was einer Rührenergie von ca. 1150 $W/m^3$ ensprach.

| Reaktionszeit | Extinktion | Conversion |
|---|---|---|
| 0 | | 0 |
| 30 | 0,499 | 80 |
| 60 | 0,1 | >95 |
| 90 | | >98 |

Reaktion nach 1,5 h beendet.

Aufarbeitung:

150 ml des Reaktionsgemischs wurden mit 75 ml MTBE geschüttelt und durch eine mit Glaswolle befüllte Säule (Höhe 9 cm, Durchmesser 3 cm) mit geringem Druck geschickt. Der Ablauf bestand aus einer blanken organischen und einer gelben, leicht getrübten wäßrigen Phase. Anschließend wurden durch dieselbe Säule noch 300 ml unverdünnten Reaktionsgemisches geschickt, wobei ebenfalls eine saubere Phasentrennung erreicht wurde. Geringe Mengen Feststoffes reicherten sich in der Säule an und wurden mit Wasser und MTBE entfernt. Danach konnte die Säule wieder zur Phasentrennung verwendet werden.
Die MTBE-Phasen wurden gesammelt und einrotiert:
Rückstand: (S)-3-Phenoxybenzaldehyd-cyanhydrin (SCMB) 52,7 g (92 %)

Beispiel 3

13,8 g (0,28 mol) NaCN wurden in 12 ml demineralisiertem Wasser gelöst, in einen Rundkolben eingebracht und mit Wasser (auf 90 ml) nachgespült. Unter Eiskühlung wurde 10 % $H_2SO_4$ aus einem Tropftrichter zugegeben, sodaß ein pH Wert von 5,6 erreicht wurde. Die so erhaltene Mischung wurde in ein 750 ml Doppelmantelgefäß mit 6-Blattrührer eingebracht. 26,4 ml Enzym (Hnl aus Pichia pastoris Überstand der Zentrifugation des unverdünnten Zellysates, 850 IU/ml) wurden mit Wasser auf 130 ml verdünnt und ebenfalls in den Reaktor eingebracht. Der pH-Wert des Reaktionsgemisches wurde auf 5,5 nachjustiert, 180 ml MTBE und 31 g (0,156 mol) m-Phenoxybenzaldehyd zugegeben und die Reaktion gestartet. Dabei wurde mit 500 rpm gerührt (3,8 $kW/m^3$), wobei eine Emulsion entstand. Der Reaktionsverlauf wurde wieder photometrisch verfolgt.

| Reaktionszeit | Extinktion | Conversion |
|---|---|---|
| 0 | | |
| 60 | 0,129 | ca. 70 |
| 120 | 0,079 | ca. 85 |
| 180 | 0,060 | ca. 90 |

Beispiel 4

Analog Beispiel 3 wurden 6,9 g (0,141 mol) NaCN, 15,5 g (0,0781 mol) m-Phenoxybenzaldehyd, 26,4 ml Enzym (850 IU/ml) in 45 ml MTBE und 16,1 ml Wasser bei pH 5,6 (eingestellt mit 10 %iger $H_2SO_4$) in einem 150 ml Doppelmantelgefäß mit 6-Blattrührer umgesetzt. Das Reaktionsgemisch wurde derart gerührt, daß eine Emulsion entstand. Die Rührerdrehzahl betrug anfangs 1000 rpm. Aus den Rührerdimensionen wurde errechnet, daß die Nennleistung der Reaktionsanlage erst bei 1860 rpm erreicht ist, sodaß nach 2,5 h die Drehzahl auf diesen Wert erhöht wurde.

| Reaktionszeit | Extinktion | Conversion |
|---|---|---|
| 0 | | |
| 60 | 0,49 | |
| 120 | 0,36 | 60 - 65 |
| 180 | 0,20 | ca. 80 |
| 240 | 0,07 | ca. 90 - 95 |

Durch die Steigerung der Rührerdrehzahl nach 150 min wurde die Reaktion deutlich beschleunigt.

Beispiel 5

Analog Beispiel 4 wurden 13,8 g (0,28 mol) NaCN, 31 g m-Phenoxybenzaldehyd, 26,4 ml Enzym in 45 ml MTBE und 220 ml Wasser bei pH 5,6 (eingestellt mit 10 %iger $H_2SO_4$) bei 15°C in einem 750 ml Doppelmantelgefäß mit 6-Blattrührer umgesetzt. Wiederum wurde so schnell gerührt, daß eine Emulsion entstand. Die Rührerdrehzahl betrug 500 rpm (5,1 kW/m³, korrigiert auf tatsächliches Reaktionsvolumen).

| Reaktionszeit | Extinktion | Conversion |
|---|---|---|
| 0 | | |
| 90 | 0,20 | ca. 70 |
| 165 | 0,047 | ca. 90 |
| 210 | 0,041 | ca. 95 |

Die Aufarbeitung erfolgte durch Durchleiten durch eine Glassäule

Das Produkt wurde durch Abdestillieren des Lösungsmittels rein erhalten. Eine analytische Probe wurde gezogen und auf übliche Art undWeise durch Reaktion mit Acetylchlorid in Dichlormethan acetyliert und sein Restaldehydgehalt und sein Enantiomerenüberschuß (ee) durch Chromatographie an einer chiralen Gaschromatographiesäule bestimmt:

GC-Analytik:
Umsatz: 97 %
e.e. 98 %

Beispiel 6

88 ml Enzym (850 U/ml) wurden mit 112 ml Wasser gemischt, mittels 0,1 %iger Citronensäure auf pH 5,5 gestellt und in ein 750 ml Doppelmantelgefäß mit 6-Blattrührer eingebracht. 50 g (0,252 mol) m-Phenoxybenzaldehyd wurden in 150 ml MTBE gelöst und der Enzymlösung zugegeben. Das Reaktionsgemisch wurde auf 15°C gebracht und mit 500 rpm (6kW/m³) gerührt, sodaß eine Emulsion vorlag. Anschließend wurden 12,3 g (0,454 mol) HCN über einen Tropftrichter innerhalb 30 min zugegeben. Der Reaktionsverlauf wurde wiederum photometrisch verfolgt.

| Reaktionszeit | Extinktion | Conversion |
|---|---|---|
| 0 | | |
| 30 | 0,795 | ca. 30 (Ende des Zutropfens) |
| 90 | 0,227 | ca. 75 |
| 150 | 0,079 | ca. 92 |
| 180 | 0,057 | ca. 95 |
| 240 | | ca. 98 (DC) |

Das Reaktionsgemisch wurde durch Phasentrennung an einer mit Glaswolle befüllten Säule aufgearbeitet.
GC Analyse: Umsatz 98 %
e.e. 99 %

Beispiel 7

4 g native (S)-Hydroxynitril-Lyase (Hnl) aus Hevea brasiliensis wurden in 100 ml Na-Citratpuffer (5mMol) aufgeschlemmt, 2,5 h bei Raumtemperatur gerührt, zen trifugiert und der Überstand auf 10 ml am Rotavapor (30°C, 1mbar) eingeengt.

Nach Messung der Aktivität (348 IU/g) wurde das Enzym in einen 25 ml Rundkolben überführt, 1 g (5mmol) m-Phenoxybenzaldehyd und 1,5 ml tert. Butylmethylether (MTBE) zugegeben.

Das so erhaltene Reaktionsgemisch wurde sodann bei 0 - 5 °C solange mit einer Rührenergie von etwa 1KW/m$^3$ gerührt (Magnetrührer), bis eine Emulsion entstand. Anschließend wurden 0,3 ml HCN (7,7 mmol) rasch zugetropft und 23 h bei 0 - 5°C mit unverminderter Rührenergie weitergerührt.

Der Reaktionsverlauf wurde mittels IP-Kontrolle über Abnahme des m-Phenoxybenzaldehydgehaltes (photometrische Messung bei 304 nm) verfolgt.

Die Reaktionslösung wurde nach beendeter Reaktion zentrifugiert, das Enzym abgetrennt und die verbleibende Lösung mit 2,5 ml MTBE verdünnt, geschüttelt und abermals zentrifugiert.

Als Rückstand in der organischen Phase verblieb (S)-3-Phenoxybenzaldehydcyanhydrin. (e.e = 90,6 %, Gehalt 91,4 %) (gaschromatographisch bestimmt)

Der Umsatz betrug nach 23 h 92,7 %.

Vergleichsversuch

Analog Tetrahedron, Vol. 52, No. 23, (1996) pp.7833-7840 wurde (S)-3-Phenoxybenzaldehyd durch Reaktion von m-Phenoxybenzaldehyd mit KCN in einem wäßrigen Citronesäurepuffer (pH - 4.0 - 4,5) (HCN wird in situ gebildet) unter Verwendung von Hnl aus Pichia pastoris hergestellt. Die Rührenergie betrug dabei etwa 100 W/m$^3$.

(S)-3-Phenoxybenzaldehyd wurde dabei zwar in einer optischen Reinheit von e.e= 99 %, jedoch mit einer Ausbeute von lediglich 9 % erhalten.

Beispiel 8:

45,6 g (0,23 mol) m-Phenoxybenzaldehyd in 12,5 ml MTBE, 12,5 ml Toluol und 52,9 ml dest. $H_2O$ und 12,5 ml Enzym (Hnl aus Pichia pastoris, 5,2 kU/ml) wurden in eine inertisierte 100 ml Schmizoapparatur mit Strombrechern, Perfusorpumpe, Kühler, Thermostat und KPG-Rührer (3 cm) chargiert und das so erhaltene Reaktionsgemisch auf ca. 20°C temperiert.

Das Reaktionsgemisch wurde, zur Erzeugung einer Emulsion mit 950 rpm (3,2 kW/m$^3$ Rührenergie) gerührt.

Anschließend, nach Erhalt einer Emulsion wurden binnen 60 min 12,6 m Blausäure (freigesetzt aus NaCN mit $H_2SO_4$) kontinuierlich zudosiert.

Die Emulsion wurde 2,5 h durch intensives Rühren (950 rpm) aufrechterhalten. Der Reaktionsverlauf wurde mittels IP-Kontrolle über die Abnahme des Aldehydgehaltes verfolgt.

| Reaktionszeit | Extinktion | Aldehydgehalt |
|---|---|---|
| Zutropfende(1h) | 0,978 | > 30 % |
| 1,5 h | 0,396 | 10,3 % |
| 2 h | 0,213 | 5,6 % |
| 2,5 h | 0,136 | 3,1 % |

Zur Aufarbeitung der Reaktionslösung wurden weitere 25 ml MTBE und 25 ml Toluol zugesetzt und 20 min gerührt.

Das Reaktionsgemisch wurde über Nacht bei Raumtemperatur stehengelassen. Eine IP-Kontrolle nach 15 h Stehzeit ergab einen Aldehydgehalt von 1,8 %.

Die Reaktionslösung wurde sodann 1 mal 30 min bei 3000 U/min zentrifugiert, die oben liegende organische Phase abgesaugt und in einen 250 ml Rundkolben überführt. (org. Phase 1 : 83,55 g). Die Wasserphase wurde in den Reaktor rückgeführt, weitere 25 ml MTBE und 25 ml Toluol zugesetzt und 20 min bei 900 U/min gerührt. Anschließend wurde das Gemisch 30 min zentrifugiert.

Als Rückstand in den vereinigten organischen Phasen verblieb SCMB (e.e 98 %, Umsatz: 92 %

Beispiel 9: (S)-4-Fluor-3-Phenoxybenzaldehydcyanhydrin

Analog Bsp. 8 wurden 10,8 g (0,05 mol) 4-Fluor-3-phenoxybenzaldehyd in 40 ml MTBE und 20 ml $H_2O$ mit 15

ml Enzym /Hnl aus Pichia pastoris, 5,2 kU/ml) mit 8 ml HCN (freigesetzt aus NaCN mittels $H_2SO_4$) bei 10 - 15°C umgesetzt. Die Blausäure wurde innerhalb 30 min kontinuierlich zudosiert. Die Rührerdrehzahl, die zur Erzielung einer Emulsion eingestellt wurde, betrug 950 rpm.

| Reaktionszeit Dosierende | Aldehydgehalt noch keine Reaktion |
|---|---|
| 1 h | > 50 % |
| 4 h | > 30 % |

Nach 4 h wurde die Rührerdrehzahl auf 500 U/min reduziert und das Reaktionsgemisch 15 h bei 12 - 15°C gerührt. Nach insgesamt 20 h betrug der Aldehydgehalt > 5,0 %.
Die Reaktionslösung wurde in einen Meßzylinder abgelassen (Ausbeute 68,72 g) und anschließend 1 mal 20 min bei 3000 U/min zentrifugiert. Die organische Phase wurde abgezogen (org. Phase 1), die Wasserphase wieder in den Reaktor überführt mit 20 ml MTBE versetzt und 20 min bei 900 U/min gerührt. Anschließend wurde wiederum 20 min zentrifugiert, die organische Phase abgezogen und mit org. Phase 1 vereinigt.
Die vereinigten organischen Phasen wurden sodann einrotiert.
Ausbeute: 11,45 (S)-4-Fluor-3-phenoxybenzaldehydcyanhydrin
(Theorie: 12,11 g) 94,55 %,
e.e.: 95,43 %.

Beispiel 10: (S)-3,4-Difluorbenzaldehydcyanhydrin

Analog Beispiel 8 wurden 8,2 g (0,05 mol) 3,4-Difluorbenzaldehyd in 40 ml MTBE und 20 ml $H_2O$ und 15 ml Enzym (Hnl aus Pichia pastoris, 5,2 kU/ml) mit 9 ml Blausäure (freigesetzt aus NaCN) bei 10 - 15°C umgesetzt. Die Rührerdrehzahl betrug wiederum 950 rpm.
Die Blausäure wurde diesmal auf einmal zugegeben. Der Reaktionsverlauf wurde mittels IP-Kontrolle verfolgt.
Nach 3,5 h Reaktionszeit war kaum noch Aldehyd nachweisbar nach 4,5 h war keine weitere Änderung des Aldehydgehaltes zu erkennen.
Die Reaktionslösung (62,5 g) wurde analog Beispiel 9 aufgearbeitet.
Ausbeute: 8,25 g (97,74 %) (S)-3,4-Difluorbenzaldehydcyanhydrin
(Theorie 8,44 g)
e.e.: 95,61 %

Beispiel: 11

8 mmol 3-Methyl-2-butanon (860 μl) wurden in 2,4 ml Methyl-t-butylether gelöst und auf 0°C abgekühlt. Nach Zugabe von 750 IU wäßrige (Hnl aus Pichia pastoris) (das Verhältnis org. Phase/wäßr. Phase betrug 0,75 : 1,0), die mit Citronensäure auf pH = 4,0 eingestellt wurde, wurde das Reaktionsgefäß druckdicht verschlossen und gerührt, sodaß eine Emulsion entstand. Bei 0°C wurden 40 mmol (1,52 ml) Blausäure zugegeben und danach wieder gerührt. Der Reaktionsverlauf wurde durch die Abnahme der C = O-Bande (1720 $cm^{-1}$) IR-spektroskopisch verfolgt. Das 3-Methyl-2-butanon war nach 2 Minuten vollständig umgesetzt.
Die Phasen der Reaktionslösung wurden getrennt, die wäßrige Phase mehrmals mit MTBE behandelt und die so erhaltenen MTBE-Lösungen vereinigt. Die organische Phase wurde über wasserfreiem Natriumsulfat getrocknet und das Lösungsmittel bei 40°C und 20 mbar abdestilliert.
Als Rückstand verblieb (S)-2-Hydroxy-2,3-dimethylbutannitril.
e.e. = 98 % (gaschromatographisch bestimmt)
Der Umsatz betrug 99 %.

Beispiel: 12

1250 μl 4-Methyl-2-pentanon (10 mmol) wurden in 3 ml Methyl-t-butylether unter Rührung gelöst und auf 0°C abgekühlt. Dazu wurden 3,9 ml (585 UI/mmol) wäßrige Enzymlösung (Hnl aus Pichia pastoris) zugegeben. Die Enzymlösung wurde vorher mit Citronensäure auf pH = 4,5 eingestellt. Es wurde 5 - 10 Minuten gerührt. Nach Entstehen einer Emulsion wurden 50 mmol (1,9 ml) wasserfreie Blausäure rasch auf einmal zugegeben, das Reaktionsgefäß druckdicht verschlossen und 5 Minuten bei 0°C gerührt. Anschließend wurden die Phasen der Reaktionslösung getrennt, die wäßrige Phase mehrmals mit Methyl-t-butylether behandelt und die so erhaltenen MTBE-Lösungen vereinigt. Es wurde über wasserfreiem Natriumsulfat getrocknet und das Lösungsmittel unter vermindertem Druck entfernt.
Als Rückstand verblieb (S)-2-Hydroxy-2,4-dimethylpentannitril.

Die gaschromatographische Analyse ergab einen Enantiomerenüberschuß von > 99 % und die IR-Spektroskopie ergab einen Umsatz von 86 %.

Beispiel 13

4,6 mmol 2-Pentanon (493 µl) wurden in 2,4 ml Methyl-t-butylether bei 0°C unter Rührung gelöst. 3,2 ml Enzymlösung (Hnl aus Pichia pastoris, 1050 UI/mmol) wurden mit der Citronensäure auf pH = 4,0 eingestellt und erst dann zugegeben. Danach wurde 5 bis 10 Minuten gerührt und in die entstandene Emulsion 25 mmol (973 µl) wasserfreie Blausäure zugegeben.

Das Reaktionsgefäß wurde druckdicht verschlossen und es wurde 5 Minuten bei 0°C gerührt (1000 U/min). Der Reaktionsverlauf wurde durch Abnahme der C=O-Bande (1720 cm$^{-1}$) mit Hilfe IR-Spektroskopie verfolgt. Laut IR-Spektroskopie war der Umsatz nach dieser Zeit vollständig. Die Phasen der Reaktionslösung wurden voneinander getrennt, die wäßrige Phase mehrmals mit MTBE behandelt und die so erhaltenen MTBE-Lösungen vereinigt und der Reaktionslösung zugegeben. Die organische Phase wurde über wasserfreiem Natriumsulfat getrocknet und das Lösungsmittel bei 40°C und 20 mbar abdestilliert.

Als Rückstand verblieb (S)-2-Hydroxy-2-methylpentannitril.

e.e. = 74 % (gaschromatographisch bestimmt).

Beispiel 14

1210 µl 2-Hexanon (10 mmol) wurden in 4 ml Methyl-t-butylether unter Rührung gelöst und auf 0°C abgekühlt. Nach Zugabe von 3,9 ml wäßriger Enzymlösung mit pH = 4,0 (Hnl aus Pichia pastoris, 585 Ul/mmol) wurde das Reaktionsgefäß druckdicht verschlossen und bis zur Emulsionsentstehung gerührt.

Dazu wurden 50 mmol (1,9 ml) wasserfreie Blausäure rasch auf einmal zugegeben und danach 5 Minuten bei 0°C gerührt. Die Reaktionsphasen wurden voneinander getrennt, die wäßrige Phase mehrmals mit Methyl-t-butylether behandelt und die vereinigten MTBE-Lösungen über wasserfreiem Natriumsulfat getrocknet, das Lösungsmittel wurde unter vermindertem Druck entfernt.

Als Rückstand verblieb (S)-2-Hydroxy-2-methylhexannitril.

Die gaschromatographische Analyse ergab einen Enantiomerenüberschuß von 98,5 % und einen Umsatz von 58,5 %.

Beispiel 15

5 mmol Acetophenon (580 µl) wurden in 1,9 ml Methyl-t-butylether gelöst und auf 0°C abgekühlt. 2,5 ml Enzymlösung (Hnl aus Pichia pastoris, 750 Ul/mmol) wurden mit Citronensäure auf pH = 3,75 eingestellt und zum Reaktionsgemisch gegeben. Danach wurde 5 bis 10 Minuten gerührt. In die entstandene Emulsion wurden 25 mmol (973 µl) wasserfreie Blausäure gegeben. Anschließend wurde das Reaktionsgefäß druckdicht verschlossen und es wurde 5 Minuten bei 0°C gerührt. Der Reaktionsverlauf wurde IR-spektroskopisch verfolgt. Die Phasen der Reaktionslösung wurden getrennt. Die wäßrige Phase mehrmals mit Methyl-t-butylether behandelt und die MTBE-Lösungen vereinigt. Die organische Phase wurde über wasserfreiem Natriumsulfat getrocknet und das Lösungsmittel unter vermindertem Druck entfernt.

Als Rückstand verblieb (S)-2-Hydroxy-2-methylphenylacetonitril.

e.e. = 99 %

IR-Spektroskopie ergab einen Umsatz von 38 %.

Beispiel 16

580 µl Acetophenon (5 mmol) wurden in 1,9 ml Diisopropylether unter Rührung gelöst und auf 0°C abgekühlt. Dazu wurden 2,5 ml (750 Ul/mmol) wäßrige Enzymlösung (Hnl aus Pichia pastoris) zugegeben, wobei die Enzymlösung vorher mit Citronensäure auf pH = 5,0 eingestellt wurde. Es wurde 5 - 10 Minuten gerührt und nach Entstehen einer Emulsion wurden 25 mmol (0,95 ml) wasserfreie Blausäure rasch auf einmal zugegeben. Das Reaktionsgefäß wurde druckdicht verschlossen und die Reaktionslösung wurde weitere 5 Minuten bei 0°C gerührt. Die Phasen der Reaktionslösung wurden getrennt, die wäßrige Phase mehrmals mit Methyl-t-butylether behandelt und die so erhaltenen MTBE-Lösungen vereinigt. Die organische Phase wurde über wasserfreiem Natriumsulfat getrocknet und das Lösungsmittel unter vermindertem Druck entfernt.

Als Rückstand verblieb 2-Hydroxy-2-methylphenylacetonitril.

Die gaschromatographische Analyse ergab einen Enantiomerenüberschuß von 99 % und die IR-Spektroskopie ergab einen Umsatz von 40 %.

**Patentansprüche**

1. Verfahren zur Herstellung der (S)-Enantiomeren von optisch aktiven Cyanhydrinen durch Umsetzung eines Aldehyds oder eines Ketons mit einem Cyanidgruppendonor in Anwesenheit einer nativen oder einer rekombinanten (S)-Hydroxynitrillyase, **dadurch gekennzeichnet, daß** ein Reaktionsgemisch aus

   a) einem in einem organischen, mit Wasser nicht oder geringfügig mischbaren Verdünnungsmittel gelösten Aldehyd oder Keton,
   b) einer wäßrigen (S)-Hydroxynitrillyaselösung und
   c) einem Cyanidgruppendonor
   mit einer Rührenergie über 500 W/m$^3$, daß eine Emulsion entsteht, die bis zum Reaktionsende durch Rühren aufrecht erhalten wird, worauf nach beendeter Reaktion das entsprechende (S)-Cyanhydrin aus dem Reaktionsgemisch durch Phasentrennung isoliert wird.

2. Verfahren nach Anspruch 1, **dadurch gekennzeichnet, daß** ein aliphatischer, aromatischer oder heteroaromatischer Aldehyd oder ein unsymmetrisches Keton umgesetzt wird.

3. Verfahren nach Anspruch 1, **dadurch gekennzeichnet, daß** als Cyanidgruppendonor Blausäure zugesetzt wird.

4. Verfahren nach Anspruch 1, **dadurch gekennzeichnet, daß** als Hydroxynitrillyase eine native (S)-Hydroxynitrillyase aus Manihot esculenta oder Hevea brasiliensis oder eine davon abgeleitete rekombinante (S)-Hydroxynitrillyase hergestellt aus Pichia pastoris, Saccharomyces cerevisiae eingesetzt wird.

5. Verfahren nach Anspruch 1, **dadurch gekennzeichnet, daß** als Verdünnungsmittel mit Wasser nicht oder geringfügig mischbare aliphatische oder aromatische Kohlenwasserstoffe, die gegebenenfalls halogeniert sind, Alkohole, Ether oder Ester oder Gemische verwendet werden.

6. Verfahren nach Anspruch 1, **dadurch gekennzeichnet, daß** als Verdünnungsmittel Methyl-tert. Butylether, Diisopropylether, Dibutylether, Ethylacetat oder ein Gemisch aus Methyltert. Butylether und Toluol verwendet wird.

7. Verfahren nach Anspruch 1, **dadurch gekennzeichnet, daß** die Reaktionstemperatur 0°C bis 30°C beträgt.


**Claims**

1. Process for the preparation of the (S)-enantiomers of optically active cyanohydrins by reaction of an aldehyde or of a ketone with a cyanide group donor in the presence of a native or of a recoinbinant (S)-hydroxynitrile lyase, **characterized in that** a reaction mixture of

   a) an aldehyde or ketone dissolved in an organic, water-immiscible or slightly miscible diluent,
   b) an aqueous (S)-hydroxynitrile lyase solution and
   c) a cyanide group donor

   is stirred with a stirring energy above 500 W/m$^3$, such that an emulsion is formed, which is maintained up to the end of the reaction by stirring, after which the corresponding (S)-cyanohydrin is isolated from the reaction mixture by phase separation after reaction is complete.

2. Process according to Claim 1, **characterized in that** an aliphatic, aromatic or heteroaromatic aldehyde or an unsymmetrical ketone is reacted.

3. Process according to Claim 1, **characterized in that** the cyanide group donor added is hydrocyanic acid.

4. Process according to Claim 1, **characterized in that** the hydroxynitrile lyase employed is a native (S)-hydroxynitrile lyase from Manihot esculenta or Hevea brasiliensis or a recombinant (S)-hydroxynitrile lyase derived therefrom, prepared from Pichia pastoris or Saccharomyces cerevisiae.

5. Process according to Claim 1, **characterized in that** the diluents used are water-immiscible or slightly miscible aliphatic or aromatic hydrocarbons which are optionally halogenated, alcohols, ethers or esters or mixtures.

6. Process according to Claim 1, **characterized in that** the diluent used is methyl tert-butyl ether, diisopropyl ether, dibutyl ether, ethyl acetate or a mixture of methyl tert-butyl ether and toluene.

7. Process according to Claim 1, **characterized in that** the reaction temperature is 0°C to 30°C.

**Revendications**

1. Procédé de préparation d'énantiomères (S) de cyanohydrines optiquement actives par réaction d'un aldéhyde ou d'une cétone avec un donneur de groupes cyanures en présence d'une (S)-hydroxynitrile lyase native ou recombinante, **caractérisé en ce qu'**un mélange réactionnel constitué de

   a) un aldéhyde ou une cétone dissous dans un agent de dilution organique non miscible ou peu miscible à l'eau,
   b) une solution aqueuse de (S)-hydroxynitrile lyase et
   c) un donneur de groupes cyanure
   est agité avec une énergie de brassage de plus de 500 W/m$^3$, de manière qu'il se forme une émulsion, qui est maintenue par brassage jusqu'à la fin de la réaction, après quoi, une fois la réaction terminée, la (S)-cyanohydrine correspondante est isolée du mélange réactionnel par séparation de phases.

2. Procédé selon la revendication 1, **caractérisé en ce que** l'on fait réagir un aldéhyde aliphatique, aromatique ou hétéroaromatique ou une cétone asymétrique.

3. Procédé selon la revendication 1, **caractérisé en ce que** l'on utilise de l'acide cyanhydrique en tant que donner de groupes cyanure.

4. Procédé selon la revendication 1, **caractérisé en ce que** l'on met en oeuvre en tant qu'hydroxynitrile lyase une (S)-hydroxynitrile lyase native provenant de Manihot esculenta ou Hevea brasiliensis ou une (S)-hydroxynitrile lyase recombinante qui en est dérivée préparée à partir de Pichia pastoris, Saccharomyces cerevisiae.

5. Procédé selon la revendication 1, **caractérisé en ce que** l'on utilise en tant qu'agent de dilution des hydrocarbures aliphatiques ou aromatiques non miscibles ou peu miscibles à l'eau, qui sont le cas échéant halogénés, des alcools, des éthers ou des esters, ou leurs mélanges.

6. Procédé selon la revendication 1, **caractérisé en ce que** l'on utilise en tant qu'agent de dilution de l'éther méthyl-tert.-butylique, de l'éther diisopropylique, de l'éther dibutylique, de l'acétate d'éthyle ou un mélange d'éther méthyl-tert.-butylique et de toluène.

7. Procédé selon la revendication 1, **caractérisé en ce que** la température de réaction est de 0°C à 30°C.